Europäisches Patentamt

European Patent Office

Office européen des brevets

(11) Publication number: **0 102 073**
A2

# (12) EUROPEAN PATENT APPLICATION

(21) Application number: **83108442.1**

(22) Date of filing: **26.08.83**

(51) Int. Cl.³: **A 61 B 5/14**

(30) Priority: **27.08.82 DK 3853/82**

(43) Date of publication of application:
**07.03.84 Bulletin 84/10**

(84) Designated Contracting States:
**AT BE CH DE FR GB IT LI LU NL SE**

(71) Applicant: **RADIOMETER A/S**
**Emdrupvej 72**
**DK-2400 Copenhagen NV(DK)**

(72) Inventor: **Andersen, Jorgen**
**Dyrespringvej 3**
**DK-2730 Herlev(DK)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath**
**Maximilianstrasse 58**
**D-8000 München 22(DE)**

(54) A syringe for collecting a blood sample.

(57) A syringe for collecting blood samples, especially arterial blood samples, comprises a syringe cylinder (11) and a piston assembly (12) displaceably arranged therein. The piston assembly defines a venting passage (25, 37, 31) through which gas or air may escape when arterial blood flows into the cylinder space (17) under the influence of the arterial blood pressure. The venting passage is controlled by valve means (23, 31), which may selectively and reversably be opened and closed independently of axial displacement of the piston assembly.

The valve means may, for example, be operated by rotating a piston rod through a predetermined angle or by means of a valve actuating lever (35), which is biased towards a position in which the valve means are closed, and which may be tilted to an open position by depressing a finger grip (36) positioned at the free outer end of the piston rod (32).

Fig. 1

Fig. 2

EP 0 102 073 A2

Croydon Printing Company Ltd.

# A SYRINGE FOR COLLECTING A BLOOD SAMPLE

The present invention relates to a syringe for collecting a blood sample from a donor's vessel and of the type comprising a syringe cylinder and a piston member, which defines therein a blood collecting chamber vented to the atmosphere.

The blood parameters determined by blood gas analysis, such as the partial pressure of oxygen ($pO_2$), the partial pressure of carbon dioxide ($pCO_2$), and the acidity (pH), may be influenced and changed when the blood sample comes into contact with the ambient atmosphere, and, consequently, it is necessary to take special measures in order to avoid or reduce such contact.

Such blood collecting syringes, wherein the blood collecting chamber is vented to the atmosphere through a venting passage while a blood sample is collected, and wherein the venting passage may be closed when a suitable amount of blood has been collected in the syringe, are disclosed for example in US Patents Nos. 3,943,917, 3,960,139, 3,978,846, 4,133,304, 4,206,768, 4,257,426, 4,266,558, 4,266,559, 4,299,238, 4,327,745, 4,340,067, 4,361,155 and 4,373,535, German Offenlegungsschrift No. 3,041,563, PCT publication No. WO 81/03426, and published European patent applications Nos. 47,176 and 47,806.

The valve means of these prior art structures are adapted to close the venting passage when the blood collecting chamber has been substantially or completely filled with the blood sample. In some of the prior art structures the valve means are adapted to close the venting passage automatically, and in other structures some kind of manual operation of the valve means is necessary. In the latter known structures the valve means are normally retained in their open position so that the blood collecting chamber is vented to the atmosphere, and when the desired volume of blood has flown into the blood collecting chamber under arterial blood pressure, the operator may close the venting passage irreversibly, whereafter the collected blood sample may be expelled from the syringe cylinder, for example into a blood gas analysing apparatus, by pushing the piston of the syringe further into the syringe cylinder.

In some cases the blood pressure within the blood vessel from which a blood sample is to be collected, is insufficient to press blood through a hollow needle into a vented blood collecting chamber of a syringe. Therefore, the operator may desire to be able to selectively open and close the venting passage defined in the piston, so that it is possible to create a subatmospheric pressure in the blood collecting chamber by closing the venting passage and slowly moving the piston outwardly in relation to the syringe cylinder. In case the blood pressure in the vessel is sufficient to cause the blood to flow through a hollow needle into a vented blood collecting chamber, the operator may open the valve means and place the piston in a desired position.

US patent No. 4,299,238 discloses a syringe of the latter type, wherein the venting passage defined in the piston is automatically closed by a valve member when the operator moves the piston assembly outwards or pushes it inwards, while the venting passage is open when the valve assembly is in a "passive" position in which the operator does not exert an axially outwardly or inwardly directed force on the piston rod. While this latter prior art structure allows withdrawal of a blood sample from a blood vessel under subatmospheric pressure and discharge of the collected blood sample from the syringe cylinder under superatmospheric pressure it is subject to serious limitations.

The present invention provides a syringe of the above type with an improved versatility, and the syringe according to the invention is of the type comprising a syringe cylinder adapted to receive a hollow needle at a first end thereof, a piston member arranged axially displaceably within the cylinder with a peripheral outer surface part of the piston member in sealing engagement with the peripheral inner surface of the syringe cylinder so as to define a blood collecting chamber therein, said chamber communicating with the ambient atmosphere through a venting passage defined in the piston member, a piston rod having its inner end connected to the piston member and having it outer end extending outwardly from the syringe cylinder at a second end thereof opposite to said first end, and valve means movable by means of selectively manually operatable control means accessible at the outer end of the piston rod, between open and closed positons, in which the venting passage is open and closed, respectively, and the syringe according to the invention is characterized in that the control means are operatable independently of axial displacement of the piston member. Because the operation of the valve means of the piston according to the invention is independent on axial movements of the piston member, the valve means may be operated not only so as to withdraw a blood sample under subatmospheric pressure, but also so as to suck air into and expell air from the blood collecting chamber through the venting passage, whereby the axial position of the piston member within the syringe cylinder may be adjusted during the sample collecting procedure, if desired.

When the syringe according to the invention is used for collecting an arterial blood sample flowing into the vented blood collecting chamber under arterial blood pressure, it is important that the operator closes the valve means exactly when the blood collecting chamber has been filled, before any substantial amount of blood flows out through the venting passage defined in the piston member, because such outflowing blood may involve a risk of infection. In order to facilitate closing of the valve means in time the syringe cylinder may be made from a transparent material, and the venting passage may comprise at least one section causing a high resistance to liquid flow therethrough. The operator may then watch the high resistance section of the venting passage and close the valve means when blood starts flowing therethrough at a relatively low rate.

It is important that the operator may easily operate the control means by one hand. Thus, the control means may comprise a lever coextending with the piston rod and having its fulcrum positioned thereon. The valve means may then be opened or closed by tilting the lever in relation to the piston rod.

P&V F3564jB, KEV/IPb, 1983 08 23

The valve means may comprise a valve seat surrounding a cross-section of the venting passage, a valve member for cooperating with the valve seat and arranged on the inner end portion of said lever, and biasing means for elastically biasing the valve member into sealing engagement with the valve seat, whereby the valve means may be moved to the open position by actuation of the outer end of said lever. By this embodiment the operator needs to actuate the lever only when the valve means are to be opened.

In an alternative embodiment the valve means comprise a valve element which is rotatably mounted in the piston member and connected to the piston rod, whereby the valve means may be moved between the open and closed positions by rotating the piston rod.

The said passage section causing high flow resistance may, for example, comprise a throat or another passage length with a restricted cross-section. However, in the preferred embodiment the said passage section contains a flow restricting, porous material. The flow restricting porous material may, for example be a hydrofobic filter material, for example made from polytetrafluoro-ethylene, a fibrous material with capillary effect, which may be yarn- or thread-like materials of cellulose, viscose rayon, wool, cotton, or jute, a material which swells or stiffens when contacted by blood, for example fibrous materials of cellulose, viscose rayon, wool, cotton, jute or materials forming a gel when contacted by aqueous mediums, such as blood. Such materials may comprise starch-based materials of the type used in babies' napkins and bandages, or freeze-dried, compressed products. The porous material is preferably of a type which allows blood to pass, and which does not completely stop the flow of blood therethrough.

The passage section causing the high flow resistance is preferably positioned upstream of or at the valve means, because the operator watching the blood flowing through the said passage section may then be ready to close the valve means in time before blood has passed through the valve means.

In order further to reduce the risk of spilling blood, the venting passage may comprise a blood receiving chamber downstream of the valve means for receiving blood, which has passed the high resistance section of the venting passage.

The invention will now be further described with reference to the drawings, wherein

Fig. 1 is a side view and partially sectional view of an embodiment of the syringe according to the invention,

Fig. 2 is a side view and partially sectional view of the piston assembly of the syringe shown in Fig. 1 in an enlarged scale,

Fig. 3 is a side view and partially sectional view of a modified embodiment of the piston assembly,

Fig. 4 is a perspective view of the piston assembly shown in Fig. 3,

Fig. 5 is a perspective view and partially sectional view of an alternative embodiment of the syringe according to the invention,

Fig. 6 is an exploded perspective view of the piston assembly shown in Fig. 5,

Fig. 7 is a side view of the front portion of the piston assembly shown in Fig. 5 in another rotational position and in an enlarged scale,

Fig. 8 is a cross-sectional view along the line 8-8 in Fig. 7,

Fig. 9 is a cross-sectional view along the line 9-9 in Fig. 7,

Fig. 10 is a cross-sectional view of the piston rod along the line 10-10 in Fig. 7,

Fig. 11 is a side view and partially sectional view of the syringe shown in Fig. 5, wherein the piston rod has been rotated to a position in which a venting passage defined in the piston assembly is closed,

Fig. 12 is an axial sectional view of the syringe shown in Fig. 11 along the line 12-12 in Fig. 11 and in an enlarged scale,

Fig. 13 is a side view of the front end portion of the piston assembly of the syringe shown in Fig. 11 and in an enlarged scale,

Fig. 14 is a cross-sectional view along the line 14-14 in Fig. 13,

Fig. 15 is a cross-sectional view along the line 15-15 in Fig. 13,

Fig. 16 is a cross-sectional view along the line 16-16 in Fig. 13,

Fig. 17 is a cross-sectional view of the piston rod along the line 17-17 in Fig. 13, and

Fig. 18 is a cross-sectional view of the piston rod along the line 18-18 in Fig. 13.

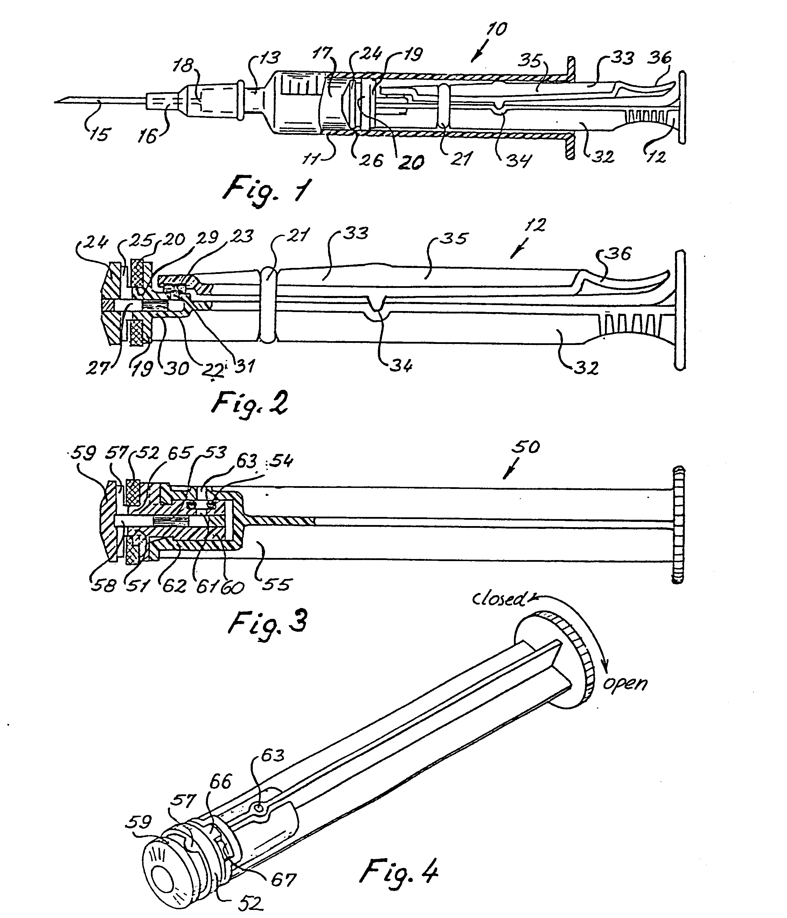

Fig. 1 shows an embodiment of the syringe according to the invention designated by the reference number 10. The syringe 10 comprises a syringe cylinder or barrel 11 and a piston or plunger assembly 12, which comprises a plurality of separate elements. The syringe cylinder 11 may, for example, be made from glass or plastic, and may be provided with a suitable gas impervious barrier layer, not shown. At its front end, the syringe cylinder 11 is provided with a hollow neck 13 for mounting a hollow needle 15 thereon. The outer peripheral surface of the hollow neck has a frusto-conical shape and is adapted to cooperate with a complementary inner frusto-conical surface of a mounting socket 16 arranged at one end of the hollow needle 15.

The syringe cylinder 11 and the piston or plunger assembly 12 displaceably arranged therein define a sample collecting chamber 17. When the hollow needle 15 is mounted on the neck 13 the inner bore of the needle 15 is in communication with the sample collecting chamber 17 through an inlet bore 18 extending axially through the neck 13 of the syringe cylinder 11. As shown in Fig. 2 the piston assembly 12 comprises a plurality of separate parts, namely a piston element 19, a sealing ring 20, an elastic rubber ring 21, a flow restricting element 22, a sealing member 23, a piston rod 32, and a valve actuating lever 35.

The front end part 24 of the piston element 19 defines an inner space 27, which is in communication with the sample collecting chamber 17 through one or more radially extending connecting passages 25. The front end part 24 of the piston element 19 has a cylindrical outer surface with a diameter, which is slightly smaller than that of the syringe cylinder 11, so as to define a narrow, annular passage 26 between the inner wall of the syringe cylinder and the outer peripheral surface of the part 24. The connecting passages 25 extend substantially radially between the hollow space 27 and the annular passage 24 and are preferably substantially uniformly spaced along the periphery of the front end part 24 of the piston element 19. The sealing ring 20 is received in an annular channel 29, which is formed in the piston element 19 adjacent to, but behind the radial connecting passages 25. The piston element 19 also comprises a rearward extension 30 formed as an integral part of the adjacent end of the piston rod, and the inner space 27 is in communication with the ambient atmosphere through an outlet opening or bore 31 formed in the wall of the extension 30.

A valve control mechanism 33 comprises the valve actuating lever 35, which is swingably mounted on the piston rod 32 at a fulcrum 34 positioned on the piston rod. The sealing member 23 is mounted on the inner end of the lever and located so that the lever may be tilted to a position in which the sealing member 23 closes the bore 31. The opposite end of the actuating lever 35 is formed as a manually operatable finger grip 36.

In the embodiment shown in Figs. 1 and 2 the sample collecting chamber is in communication with the ambient atmosphere through a venting passage formed by the narrow passage 26, the connecting passages 25, the inner space 27, and the outlet opening or bore 31. This venting passage contains the flow restricting element 22, which is arranged within the inner space 27, and which may be a porous material of the type described above.

The valve actuating lever 35 and the sealing member 23 mounted thereon cooperates with the outlet opening or bore 31 so as to form a valve, which is biased towards its closed position by means of the elastic rubber ring 21, and which is therefore normally maintained in its closed position. However, when the syringe is held in the normal oblique sampling position the operator may selectively move the valve to its open position by exerting a small pressure on the finger grip 36 by his thumb. When the finger grip is released, the valve actuating lever is automatically returned to its closed position under the bias of the rubber ring, whereby the operator may selectively open and close the venting passage during sampling independently of the possible axial movement of the piston assembly. The syringe cylinder 11 may contain dry heparine for preventing coagulation of the blood sample in a known manner.

Figs. 3 and 4 show an alternative embodiment of the piston or plunger assembly which is designated by the reference numeral 50. As best shown in Fig. 3, the piston assembly 50 comprises a hollow front piston element 51, a sealing ring 52, a flow restricting element 53, an annular sealing member 54, and a piston rod 55. The hollow front piston element 51 defines an inner space 58, which is in communication with the sample collecting chamber (not shown in Figs. 3 and 4) through one or more connecting passages 57. As in the embodiment described above the outer diameter of the front end portion of the piston element 51 is slightly smaller than the inner diameter of the associated syringe cylinder or barrel, whereby a narrow passage is defined between the inner wall of the syringe cylinder and the outer peripheral surface of the front end portion 59.

The connecting passages 57 extend substantially radially between the inner hollow space 58 and the said narrow passage and are preferably substantially uniformly spaced along the periphery of the front end portion 59 of the piston element 51.

The sealing ring 52 is received in an annular channel 65, which is formed in the piston element 51 adjacent to, but behind the radial connecting passages 57. The inner space 58 of the piston element 51 contains the flow restricting element 53 and continues into a rearward extension 60. The inner space 58 is in communication with the ambient atmosphere through an outlet opening or bore 61 formed in the walls of the extension 60.

The extension 60 is rotatably received in a cup-shaped part 62, which is formed as an integral part of the piston rod 55. An outlet opening or bore 63 is formed in the peripheral wall of the cup-shaped part 62 so that it is aligned with the outlet opening 61 of the extension 60 in one mutually rotational position of the parts 60 and 62.

As indicated in Fig. 4, the piston rod 55 and the cup-shaped part 62 integrally connected therewith may be rotated through a certain angle in relation to the piston element 51 and its extension 60. This mutual angular movement is restricted by a projection 66, which is formed on the piston element 51, and which cooperates with an angular recess or cut-out 67 formed in the free edge portion of the cup-shaped part 62.

The front piston element 51 and the cup-shaped part 62 of the piston rod 55 is made from materials with low mutual friction so that the part 62 of the piston rod 55 may easily be rotated in relation to the extension 60 of the piston element 51. The sealing ring 52 should be made from such a material that the frictional forces between the sealing ring 52 and the inner surface of the syringe cylinder exceed the frictional forces between the cup-shaped parts 62 and the extension 60 of the piston element 51, when the piston rod 55 is rotated. The piston element 51 and the sealing ring 52 mounted thereon are then kept stationary in relation to the syringe cylinder when the piston rod 55 and the cup-shaped part 62 formed thereon is rotated.

The annular sealing member 54 is arranged in a corresponding recess surrounding the outlet end of the bore 61, so that the sealing member 54 is in sealing engagement with the inner surface of the cup-shaped part 62. Thus these parts form a valve for selectively opening and closing a venting passage formed by the connecting passages 57, the inner space 58, the outlet opening or bore 61, and the bore 63. The valve may be operated by rotating the piston rod 55, and the valve is opened when the piston rod 55 is in a rotational position in which the bores 61 and 63 are aligned, while the valve is closed when the piston rod 55 is in a rotational position in which the annular sealing member 54 does not cover any part of the bore 63. Consequently, the operator may at any time open or close the venting passage by rotating the piston rod 55.

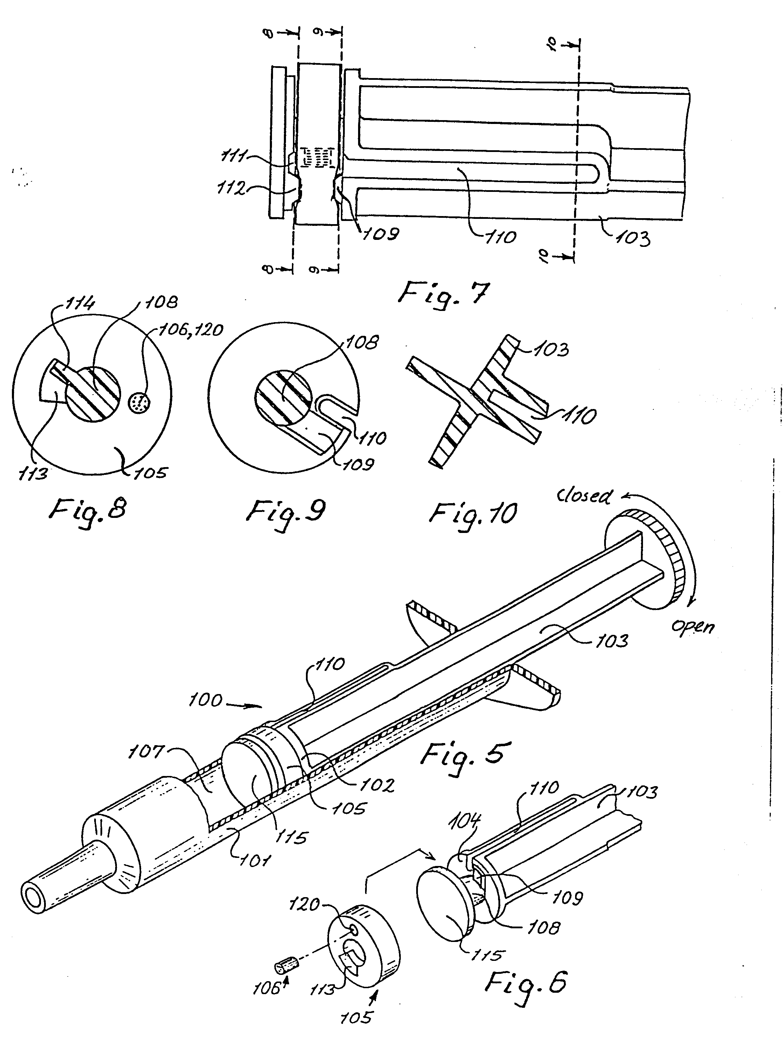

Figs. 5-18 illustrate a third embodiment of the syringe according to the invention. Like those previously described the syringe shown in Fig. 5 comprises a syringe cylinder 101 and a piston assembly 102 which define a blood collecting chamber 107 in the cylinder. The

P&V F3564jB, KEV/IPb, 1983 08 23

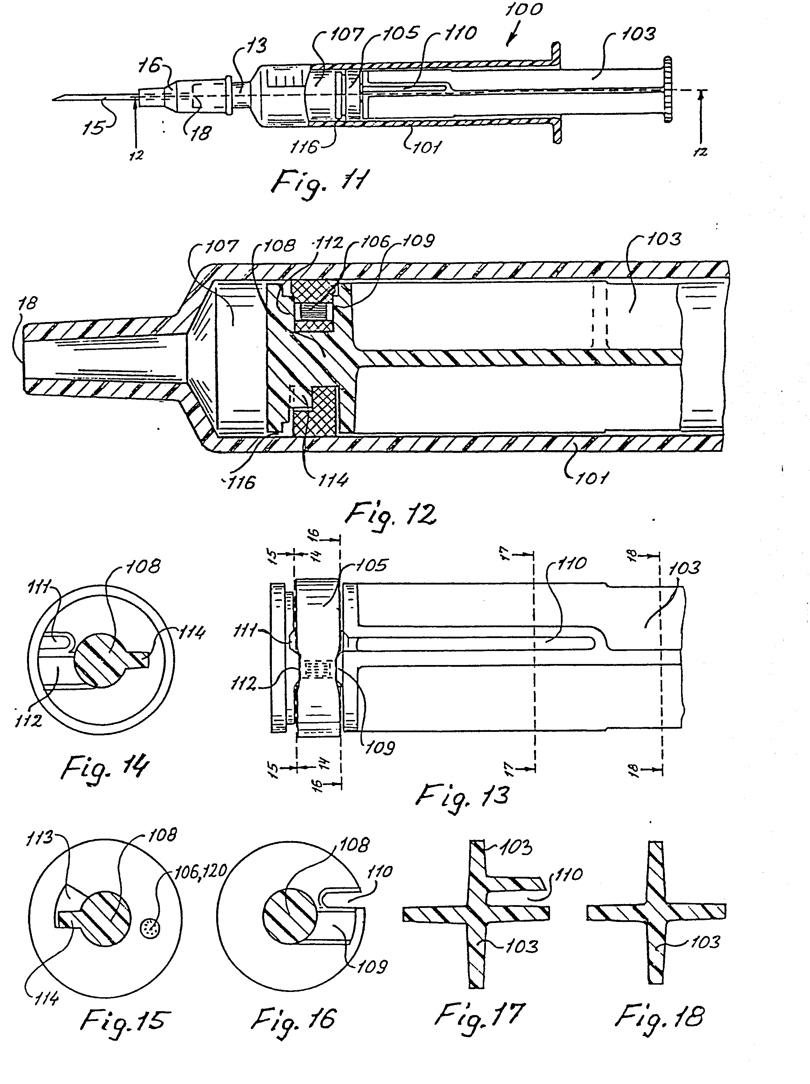

piston assembly 102 comprises valve means which, like those described in connection with Fig. 3, may selectively and reversably be opened and closed by rotative movement of a piston rod 103. As best shown in Fig. 6 the piston assembly 102 comprises a spool-shaped front portion 104, which is integrally connected to the piston rod 103, a sealing ring 105, and a flow restricting element 106. The spool-shaped front portion defines an annular channel 108 for tightly receiving the sealing ring 105 as shown in Fig. 5. Projections 109 and 112, which are shown in Figs. 6 and 14, respectively, are formed on the oppositely arranged, radially extending side surfaces of the annular channel 108. The flow restricting element 106 is received in an axial, through-going bore 120 defined in the sealing ring 105 as shown in Fig. 6.

The outer diameter of the forward flange 115 of the spool-shaped front portion 104 is slightly smaller than the diameter of the inner surface of the syringe cylinder 101, so that a narrow annular passage 116 is defined between the inner cylinder wall and the flange 115 as shown in Fig. 12.

As illustrated in Fig. 5, the piston rod 103 and the spool-shaped front portion 104 may be rotated in relation to the syringe cylinder 101, and as best shown in Fig. 8 the angular movement of the piston rod is restricted by a projection 114 extending radially from the bottom surface of the channel 108 and cooperating with a sector-shaped cut-out or recess 113 defined in the sealing ring 105.

The spool-shaped portion 104 and the sealing ring 105 are made from materials having a low mutual friction, so that the piston portion 104 may be easily rotated in relation to the sealing ring 105. Furthermore, the peripheral wall of the syringe cylinder 101 is made from a material creating a relatively high friction in relation to the sealing ring 105, so that the sealing ring is retained stationary in relation to the syringe cylinder when the piston rod 103 and the spool-shaped piston portion 104 is rotated in relation to the sealing ring 105.

P&V F3564jB, KEV/IPb, 1983 08 23

The function and shape of the valve means are shown more in detail in Figs. 7-18. Figs. 7-9 show the piston assembly 102 with the valve means in an open position, so that the venting passage is open. This venting passage comprises the annular passage 116, a depression 111 formed in the channel-defining surface of the flange 115, the through-going bore 120 receiving the flow restricting element 106 and defined in the sealing ring 105, and a channel-shaped, axially extending chamber 110 formed in the piston rod 103 and communicating with the annular channel 108. Figs. 12-18 show the piston assembly 102 when the venting passage is closed by the valve means. In this position the bore 120 is located between the extensions 109 and 112, so that no communication between the depression 111 and the chamber 110 exists, and neither gas nor blood may pass through the venting passage.

When the syringe according to the invention is used for collecting a sample of arterial blood the operator holds the syringe cylinder in one hand and moves the piston assembly by the other hand to a position in which the volume of the blood collecting chamber 107 substantially corresponds to the desired volume of the blood sample. A hollow needle 15 of a well known type is now mounted on the hollow neck 13 of the syringe cylinder, whereafter the pointed end of the hollow needle is inserted into an artery. When the needle has penetrated into the artery, arterial blood flows through the hollow needle and into the blood collecting chamber 107 under the influence of the blood pressure in the artery. As blood flows into the blood collecting chamber, air or gas is expelled therefrom through the venting passage. The flow restricting element 106, which may be of the type described above, does not substantially resist gas flow through the venting passage. However, when the blood collecting chamber 107 has been filled with blood and blood starts flowing into the venting passage and through the porous element 106, the flow rate is substantially reduced, so that the operator has sufficient time to rotate the piston rod 103 so as to move the valve means to the closed position before any substantial amount of blood has passed through the element 106. Blood which might have passed the element is, however, collected in the chamber 110, so that spillage of blood and the risk of infection is prevented or substantially reduced. When the hollow needle has been removed from the syringe cylinder the collected blood sample may be expelled directly into a blood gas analysing apparatus.

It is understood that various changes and modifications of the embodiments described above with reference to the drawings may be made within the scope of the present invention. Thus, the valve means may be of any suitable type allowing selective and reversable opening and closing of the venting passage independently of the axial movement of the piston assembly.

P&V F3564jB, KEV/IPb, 1983 08 23

CLAIMS.

1. A syringe for collecting a blood sample from a donor's blood vessel and comprising a syringe cylinder (11, 101) adapted to receive a hollow needle (15) at a first end thereof, a piston member (12, 50, 102) arranged axially displaceably within the cylinder with a peripheral outer surface part of the piston member in sealing engagement with the peripheral inner surface of the syringe cylinder so as to define a blood collecting chamber (17, 107) therein, said chamber communicating with the ambient atmosphere through a venting passage (25, 27, 31; 57, 58, 61, 63; 111, 120, 110) defined in the piston member, a piston rod (32, 55, 103) having its inner end connected to the piston member and having its outer end extending outwardly from the syringe cylinder at a second end thereof opposite to said first end, and valve means (23, 31; 54, 62; 105, 110) movable by means of selectively manually operatable control means (33, 55, 103) accessible at the outer end of the piston rod, between open and closed positions, in which the venting passage is open and closed, respectively, characterized in that the control means (33, 55, 103) are operatable independently of axial displacement of the piston member (12, 50, 102).

2. A syringe according to claim 1,
characterized in that the syringe cylinder is made from a transparent material, and the venting passage comprises at least one section (22, 53, 106) causing a high resistance to liquid flow therethrough.

3. A syringe according to claim 1 or 2,
characterized in that said control means comprises a lever (35) coextending with the piston rod (32) and having its fulcrum (34) positioned thereon.

4. A syringe according to claim 3,
characterized in that the valve means comprise a valve seat surrounding a cross-section of the venting passage (25, 27, 31), a valve member (23) for cooperating with the valve seat and arranged on the inner end portion of said lever (35), and biasing means (21) for

P&V F3564jB, KEV/IPb, 1983 08 23

0102073

elastically biasing the valve member into sealing engagement with the valve seat, whereby the valve means may be moved to the open position by actuation of the outer end of said lever.

5. A syringe according to claim 4,
characterized in that said biasing means comprises an elastic ring (21) surrounding and tightly engaging with the piston rod (32) and said lever (35) at a position spaced from the fulcrum (34) of the lever.

6. A syringe according to claim 1 or 2,
characterized in that the valve means comprise a valve element (62, 104) which is rotatably mounted in relation to the piston member (50, 102) and connected to the piston rod (55, 103), whereby the valve means may be moved between the open and closed positions by rotating the piston rod.

7. A syringe according to claim 6,
characterized in that the venting passage comprises a passage section (63, 110) defined in the valve element (55, 103) so as to communicate with the venting passage defined in the piston member in a first relative rotational position of the valve element, while the venting passage in the piston member is closed by a surface part of the valve element in a second relative rotational position thereof.

8. A syringe according to claim 7,
characterized in that the piston member comprises an annular sealing ring (52, 105) rotatably received in an annular channel (65, 108) formed at the inner end portion of the piston rod (55, 103).

9. A syringe according to any of the claims 2-8,
characterized in that said passage section causing high flow resistance contains a flow restricting, porous material (22, 53, 106).

10. A syringe according to claim 9,
characterized in that the porous material is a fibrous material.

P&V F3564jB, KEV/IPb, 1983 08 23

11. A syringe according to any of the claims 8-10,
characterized in that at least part of the piston member engaging with the inner wall of the syringe cylinder is made from an elastomeric material.

12. A syringe according to any of the claims 2-11,
characterized in that the passage section (22, 53, 106) causing a high flow resistance is positoned upstream of the valve means.

13. A syringe according to any of the claims 1-12,
characterized in that the venting passage comprises a blood receiving chamber (110) downstream of the valve means for receiving blood, which has passed the high resistance section of the venting passage.

0102073

1/3

Fig. 1

Fig. 2

Fig. 3

Fig. 4

Fig. 7

Fig. 8

Fig. 9

Fig. 10

Fig. 5

Fig. 6

0102073

Fig. 11

Fig. 12

Fig. 14

Fig. 13

Fig. 15

Fig. 16

Fig. 17

Fig. 18